# EUROPÄISCHE PATENTANMELDUNG

(11) **EP 1 688 108 A2**
(43) Veröffentlichungstag der Anmeldung: **09.08.2006**
(21) Anmeldenummer: 06000556.8
(22) Anmeldetag: 12.01.2006
(51) Int. Cl.: A61F 7/02

(54) **Kühl- oder Wärmepackung für medizinische Zwecke**

(30) Priorität: 07.02.2005 CH 1862005
(71) Anmelder: Gadient, Konrad, 8501 Frauenfeld (CH)
(72) Erfinder: Gadient, Ester M., 8501 Frauenfeld (CH); Gadient, Konrad, 8501 Frauenfeld (CH)
(74) Vertreter: Römpler, Aldo

(57) **Zusammenfassung**

Eine Mehrzahl von Hohlkörpern (1) ist frei beweglich in einer Hülle angeordnet. Die Hohlkörper (1) enthalten ein Kälte oder Wärme speicherndes Medium (2). Letzteres kann dadurch nicht zu einer einzigen, die Hülle füllende Masse zusammenfrieren. Die Hülle schmiegt sich somit gerade auch im medizinisch wirksamen, eiskalten Zustand optimal an die zu kühlende Körperpartie. Die beim Patienten verwendete und mit Körper- oder Wundflüssigkeiten in Kontakt gekommene Hülle kann entsorgt werden. Die darin geschützten Hohlkörper (1) können hingegen wieder verwendet werden. Das problematische Reinigen und Sterilisieren bisheriger, mit Gel gefüllter Beutel entfällt.

## Beschreibung

Die vorliegende Erfindung betrifft eine Kühl- oder Wärmepackung für medizinische Zwecke.

Derartige Packungen sind seit vielen Jahren bekannt. Sie bestehen gewöhnlich aus einem Kunststoffbeutel, der mit einem Gel gefüllt ist. Dieser Beutel kann in einem Kühlschrank oder Gefrierfach gekühlt werden und ist dann auf die zu behandelnde Körperstelle zu legen. Um einem Kälteschaden vorzubeugen, wird der Beutel dabei meistens in ein Handtuch eingeschlagen. Beispielsweise können mit solchen Beuteln Verletzungen von Muskeln oder Gelenken behandelt werden. Durch die kühlende Wirkung werden Schwellungen und Schmerzen abgebaut. Allenfalls können dadurch auch weitere medizinische Behandlungen erleichtert werden.

Da sich der Beutel am Körper relativ rasch erwärmt, verliert er schnell seine Wirkung. Der Beutel muss also immer wieder neu gekühlt, beziehungsweise jeweils gegen einen zweiten, kühlen Beutel ausgetauscht werden. In der Praxis zeigen sich dabei entscheidende Nachteile. Aus medizinischer Sicht ist es ungünstig, dass das ursprünglich weiche Gel beim Kühlen fest wird. Dadurch ist der Beutel im gekühlten Zustand recht steif. Gerade in demjenigen Temperaturbereich, in dem der Beutel seine Wirkung entfalten sollte, passt er sich sehr schlecht dem Körper an. Beispielsweise liegt er daher nicht richtig an einem Arm oder Knie an. Das tut er erst nachdem er wieder wärmer geworden ist und nichts mehr nützt. Ein weiterer Nachteil liegt in der Handhabung der Beutel im medizinischen Betrieb eines Spitals oder einer Arztpraxis. Diese dürfen aus Gründen der Hygiene und Sterilität nicht achtlos zur Behandlung verschiedener Patienten verwendet werden. Gerade im Fall von Verletzungen, könnte der Beutel auch mit Körper- oder Wundflüssigkeiten in Kontakt kommen. Dagegen hilft auch das Einschlagen in ein Handtuch nichts. Somit ist eine wiederholte Reinigung und Sterilisation der Beutel notwendig, was einem praktischen Betriebsablauf nicht besonders förderlich ist und auch die Gefahr von Beschädigungen der Beutel erhöht. Für diesen Nachteil gibt es bisher keine praktikable Lösung. Das zuvor angesprochene Problem der schlechten Verformbarkeit des gefrorenen Beutels hat man dadurch zu lösen versucht, dass das Kältemedium nicht als homogene Masse in den Beutel gefüllt wurde, sondern in Form eines Granulats. Das brachte jedoch nicht den gewünschten Erfolg, da sich die Granulatteile beim Kühlen sofort wieder zu einer kompakten, gefrorenen Masse verbinden. Diese harte, gefrorene Masse bringt gegenüber dem bekannten Gel keinerlei Vorteile.

Auf der Grundlage dieser Erkenntnisse setzt sich die Erfindung die Aufgabe, eine Kühl- oder Wärmepackung für medizinische Zwecke zu schaffen, die sich einerseits gut den zu behandelnden Körperstellen anpasst und andererseits praktisch in der Handhabung ist.

Die erfindungsgemässe Kühl- oder Wärmepackung entspricht den kennzeichnenden Merkmalen des Patentanspruchs 1. Weitere vorteilhafte Ausbildungen des Erfindungsgedankens sind aus den abhängigen Patentansprüchen ersichtlich.

Durch die Erfindung ergeben sich bezüglich der Anwendung und Handhabung eine Reihe von Vorteilen, auf die im folgenden noch eingegangen wird. Da eine Kältepackung nicht aus einem direkt mit einem gefrorenen Gel oder einem gefrorenen Granulat gefüllten, dünnen Plastikbeutel besteht, ist auch die Gefahr von Kälteschäden am Körper deutlich verringert. Die Erfindung eignet sich darüber hinaus sehr gut auch für Wärmepackungen. Diese sind beispielsweise bei nicht entzündlichem Rheuma oder bei Gelenkschmerzen indiziert.

Nachfolgend werden bevorzugte Ausführungsbeispiele der Erfindung anhand der Zeichnung näher beschrieben.
- Fig. 1: zeigt ein erstes Beispiel eines Hohlkörpers im Schnitt;
- Fig. 2: zeigt ein zweites Beispiel eines Hohlkörpers, ebenfalls im Schnitt;
- Fig. 3: zeigt eine mit einer Vielzahl von Hohlkörpern gefüllte Hülle;
- Fig. 4: zeigt schematisch die praktische Handhabung der Kühl- oder Wärmepackung.

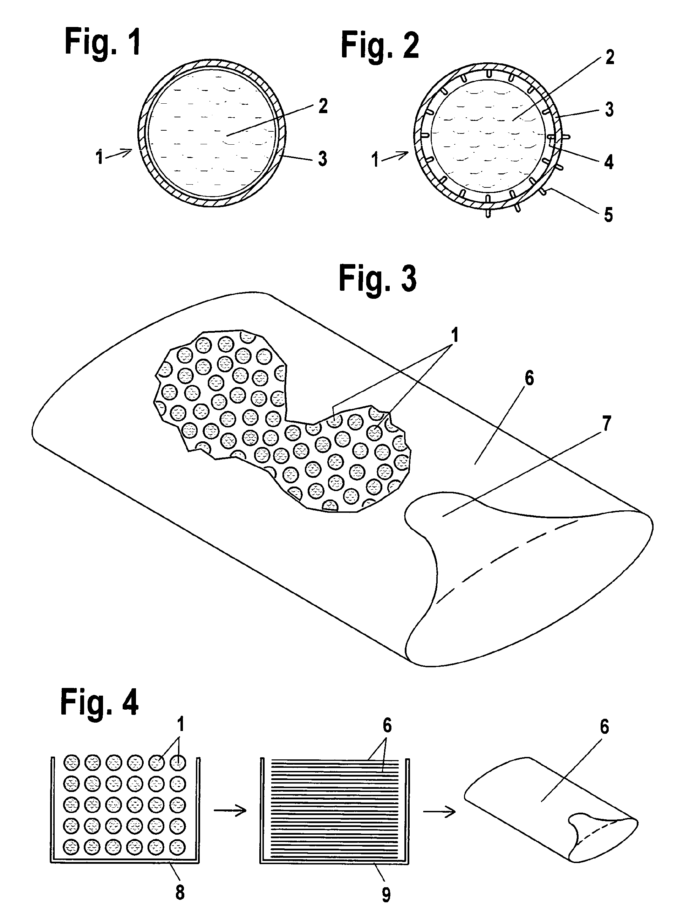

Das Kälte oder Wärme speichernde Medium 2 befindet sich in einem Hohlkörper 1 nach Fig. 1. Als Medium 2 sind alle bisher bekannten Mittel verwendbar. Beim Medium 2 kann es sich aber auch einfach nur um Wasser handeln, ohne Zusatzmittel. Chemikalien sind nicht unbedingt erforderlich, was nur als Vorteil betrachtet werden kann. Selbst im Fall von Beschädigungen der Kühl- oder Wärmepackung kann daher nichts passieren. Derartige Probleme sind indessen bei der vorliegenden Erfindung ohnehin sehr viel unwahrscheinlicher als bei herkömmlichen Gel-Beuteln.

Der Hohlkörper 1 kann aus Kunststoff bestehen. Dieser kann eine dünne Wand 3 aufweisen. Die Wand 3 kann relativ starr sein, das heisst einem Druck von innen oder aussen nur nach einem Widerstand nachgeben. da sich dies bei der Anwendung nur wenig bemerkbar macht. Alternativ kann diese Wand 3 aber auch aus einem weichen, biegsamen und dehnbaren Material gefertigt sein. Wichtig ist in beiden Fällen nur, dass der Hohlkörper 1 bei tiefen Temperaturen nicht durch das in ihm befindliche Medium 2 gesprengt werden kann. Es sei darüber hinaus ausdrücklich darauf hingewiesen, dass der Hohlkörper 1 nicht nur in der dargestellten und bevorzugten sphärischen Form denkbar ist. Theoretisch kann er jede beliebige Form aufweisen. Auch bei der Grösse, beziehungsweise dem Durchmesser des Hohlkörpers 1, liegt ein erheblicher Spielraum. Sinnvoll erscheint ein Durchmesser von 5 - 10 mm. Aber auch kleinere oder grössere Hohlkörper 1 sind möglich, bis hin zur gemeinsamen Verwendung von Hohlkörpern 1 unterschiedlicher Grösse.

Wie aus Fig. 2 ersichtlich, kann der Hohlkörper 1 auch Ausformungen 4 und/oder 5 aufweisen. Innere Ausformungen 4 können dazu dienen, einer Beschädigung der Wand 3 im Fall des sich beim Gefrieren ausdehnenden Mediums 2 vorzubeugen. Äussere Ausformungen 5 können als Abstandshalter dienen, beispielsweise um einem Kälteschock auf der Haut des Patienten vorzubeugen oder um ein Zusammenfrieren mehrerer Hohlkörper 1 zu verhindern, falls sich an deren Oberfläche Feuchtigkeit absetzen sollte. Als Beispiel sind an der Aussenseite der Wand 3 nur einige Ausformungen 5 angedeutet. Tatsächlich würden sie aber über den gesamten Umfang des Hohlkörpers 1 vorhanden sein. Im vorliegenden Fall weisen die Ausformungen 4 und 5 die Gestalt von Noppen auf. Denkbar wären aber auch andere Formen.

Zur Anwendung beim Patienten ist eine Mehrzahl vom Hohlkörpern 1 in einer Hülle 6 unterzubringen. Die Hülle 6 kann ein herkömmlicher Beutel aus dünnwandigem Kunststoff sein. Denkbar sind aber Hüllen 6 aus nahezu beliebigem Material. Auch die Form braucht nicht unbedingt rechteckig zu sein. Für bestimmte Anwendungen kann es sogar von Vorteil sein, zum Beispiel gebogene Hüllen oder solche mit Ausbuchtungen vorzusehen. Vorteilhaft ist die Hülle 6 mit einem Verschluss 7 ausgebildet, wie er in Fig. 3 als Beispiel angedeutet ist. Es ist eine Vielzahl geeigneter Verschlüsse 7 bekannt, vom Druck- oder Reissverschluss bis hin zum Klebeverschluss. Alles ist möglich, auch ein einfaches Zusammenbinden der Hülle 6. Entscheidend ist nur, dass die Hohlkörper 1 nicht herausfallen können.

Die erfindungsgemässe Kühl- oder Wärmepackung schmiegt sich optimal an die zu kühlende oder allenfalls zu wärmende Körperpartie, denn der Inhalt der Hülle 6 besteht aus einer Vielzahl von einzelnen Hohlkörpern 1. Diese sind in der Hülle 6 frei beweglich. Da sich das Kälte speichernde Medium 2 im Inneren der Hohlkörper 1 befindet, kann es beim Kühlen in einem Kühlschrank nicht zu einer einzigen, die Hülle 6 füllende Masse zusammenfrieren. Die Beweglichkeit der Hohlkörper 1 untereinander bleibt auch im gefrorenen Zustand erhalten. Die Hülle 6 lässt sich somit gerade auch im medizinisch wirksamen, eiskalten Zustand ungehindert den jeweiligen Körperrundungen anpassen. Die im Medium 2 gespeicherte Kälte wird nach und nach gleichmässig auf die durch die Hülle 6 bedeckte Körperpartie abgegeben. Im Vergleich zu den bisher bekannten Kühlpackungen wird die Kälte zudem schonend und über einen längeren Zeitraum abgegeben. Um diesen Effekt noch zu verstärken, können auch dickere Kühlpackungen verwendet werden, das heisst mit einer grösseren Anzahl an Hohlkörpern 1. Denkbar sind beispielsweise Kühlpackungen von bis zu 10 cm Dicke.

Auch bezüglich der Handhabung der Kühl- oder Wärmepackung im medizinischen Betrieb eines Spitals oder einer Arztpraxis ergeben sich erhebliche Vorteile. Die Hohlkörper 1 können in einem Gebinde 8 und die Hüllen 6 in einem separaten Gebinde 9 geliefert und bereitgestellt werden. Erst bei Bedarf wird die benötigte Menge an Hohlkörpern 1 in eine Hülle 6 gefüllt, in den Kühlschrank gelegt und schliesslich am Körper angelegt. Wie bereits erwähnt, können dabei auch Hüllen 6 unterschiedlicher Grösse und Gestalt zur Verfügung gestellt werden. Das Füllen mit unterschiedlichen Mengen an Hohlkörpern 1 ist kinderleicht, es braucht dazu kein geübtes Personal.

Die beim Patienten verwendete und mit Körperflüssigkeiten, eventuell sogar mit Blut oder Eiter in Kontakt gekommene Hülle 6 kann danach entsorgt werden. Leere Hüllen 6 brauchen kaum Platz. Die darin geschützten Hohlkörper 1 können hingegen wieder verwendet und problemlos in eine neue Hülle 6 geschüttet werden. Sie kommen immer nur mit dem Inneren einer Hülle 6 und niemals mit dem Körper des Patienten in Berührung. Das problematische Reinigen und Sterilisieren bisheriger, mit Gel gefüllter Beutel entfällt.

## Patentansprüche

1. Kühl- oder Wärmepackung für medizinische Zwecke, **gekennzeichnet durch** eine Mehrzahl von Hohlkörpern (1), die ein Kälte oder Wärme speicherndes Medium (2) enthalten, wobei diese Hohlkörper (1) ihrerseits in einer Hülle (6) angeordnet sind.

2. Kühl- oder Wärmepackung nach Anspruch 1, **dadurch gekennzeichnet, dass** die Hohlkörper (1) eine Wand (3) aus einem biegsamen und/oder dehnbaren Material aufweisen.

3. Kühl- oder Wärmepackung nach Anspruch 1 oder 2, **dadurch gekennzeichnet, dass** die Hohlkörper (1) aus Kunststoff bestehen.

4. Kühl- oder Wärmepackung nach einem der Ansprüche 1 - 3, **dadurch gekennzeichnet, dass** die Hohlkörper (1) eine sphärische Form aufweisen.

5. Kühl- oder Wärmepackung nach Anspruch 4, **dadurch gekennzeichnet, dass** die Hohlkörper (1) einen Durchmesser von 5 - 10 mm aufweisen.

6. Kühl- oder Wärmepackung nach einem der Ansprüche 1 - 5, **dadurch gekennzeichnet, dass** die Hohlkörper (1) Wasser oder ein Wasser aufweisendes Medium (2) enthalten.

7. Kühl- oder Wärmepackung nach einem der Ansprüche 1 - 6, **dadurch gekennzeichnet, dass** die Hohlkörper (1) innen und/oder an ihrem Aussenumfang Ausformungen (4, 5) aufweisen, beispielsweise Noppen.

8. Kühl- oder Wärmepackung nach einem der Ansprüche 1 - 7, **dadurch gekennzeichnet, dass** die Hohlkörper (1) in einer Hülle (6) aus einem biegsamen und/oder dehnbaren Material angeordnet sind, beispielsweise Kunststoff.

9. Kühl- oder Wärmepackung nach einem der Ansprüche 1 - 8, **dadurch gekennzeichnet, dass** die Hülle (6) mittels eines Verschlusses (7) verschliessbar ist, beispielsweise mittels eines Klebeverschlusses.

10. Kühl- oder Wärmepackung nach einem der Ansprüche 1 - 9, **dadurch gekennzeichnet, dass** die Hülle (6) eine gebogene Form und/oder mindestens eine Ausbuchtung aufweist.
